# EUROPEAN PATENT SPECIFICATION

(11) **EP 2 512 401 B1**
(45) Date of publication and mention of the grant of the patent: **06.09.2017**
(21) Application number: 10838046.0
(22) Date of filing: 04.10.2010
(51) Int. Cl.: A61K 8/02, A61K 8/44, A61K 8/891, A61K 8/34, A61Q 1/02, A61Q 1/10, A61Q 17/04, A61Q 19/00

(54) **CLEAR OR TRANSLUCENT COMPOSITION**
KLARE ODER DURCHSICHTIGE ZUSAMMENSETZUNG
COMPOSITION LIMPIDE OU TRANSLUCIDE

(30) Priority: 17.12.2009 US 640136
(43) Date of publication of application: 24.10.2012
(73) Proprietor: Avon Products, Inc., New York, NY 10017 (US)
(72) Inventor: DO, Thi, N., West Orange, NJ 07052 (US); MCNAMARA, William, E., Chester, NY 10918 (US); HALES, Kelly, Nyack, NY 10960 (US)
(74) Representative: Dr. Weitzel & Partner
(86) International application number: PCT/US2010/051295
(87) International publication number: WO 2011/075199

(56) References cited:
- WO-A1-2004/054524
- US-A1- 2002 086 039
- US-A1- 2004 229 984
- US-A1- 2006 078 581
- US-A1- 2009 280 077
- US-A1- 2009 280 077
- US-B1- 6 214 329

## Description

### TECHNICAL FIELD

The present disclosure relates to the cosmetic, cosmeceutical, and personal care arts. In particular, a combination of particular components that results in a clear or translucent composition may be processed using existing manufacturing equipment under specific conditions. The processes of the instant disclosure lead to the formation of a clear or translucent composition obtained in an unexpected and highly beneficial manner.

### BACKGROUND

Cosmetic formulations or personal care products may be prepared in a solid, semi-solid, or gel composition. For example, compressed powder sticks, gel sticks, and wax sticks are traditionally used. These formulation types have advantages in certain situations, however, each also has disadvantages. Compressed powder sticks are known to be brittle and hard, and have a tendency to leave a cosmetically-unacceptable dust upon application. While gels can provide very good aesthetic characteristics, they may be unstable due to, for example, undesirable interactions between gelling agents which are typically used to solidify such sticks and the "active" ingredient (e.g., sunscreens and antiperspirant salts). Wax-based formulations can also yield cosmetically-unacceptable products due to its hardness, greasiness, and stickiness. Another disadvantage of wax-based stick formulations is that the wax results in an opaque formulation which interferes with a colorant's true color from being presented.

Traditional clear or translucent stick compositions typically do not exist in a "free standing stick" configuration, mostly due to the fact that the composition is too soft and cannot exist without the support of a walled container. Another problem in the art is that clear stick compositions tend to sweat one or more solvents under elevated temperature conditions. Additionally, such sticks melt under high temperature conditions and potentially become cloudy when cycled between freezing conditions and ambient temperature which are typically 25°C. Generally, these elevated temperatures and/ or freezing temperatures occur during storage and the transportation of the stick product.

It is therefore an object of the disclosure to provide a clear or translucent composition, that has sufficient strength to stand freely on its own, avoid solvent from seeping out from the composition at high temperatures, and that transmits a true color.

### SUMMARY

It is one object of the disclosure to provide for a clear or translucent composition, comprising:
(a) at least two amino acid - based gelatinizing agents;
(b) a non-ionic, unsaturated fatty alcohol;
(c) an alkyl dimethicone; and
(d) an oil, a polymer, or combinations thereof,
where the composition has a dissolution temperature at or below about 100 °C and the dissolution time occurs in minutes.

Another object of the disclosure is directed to a clear or translucent composition that takes the form of a solid, a semi-solid, or a viscous gel. A further embodiment relates to the clear or translucent composition in a stick format.

It is an object of the disclosure to produce a clear or translucent stick with reproducible hardness. Without this higher stick strength, the sticks cannot be removed from the manufacturing moulds without damage.

In yet another object of the disclosure, the clear or translucent composition of the disclosure is a cosmetic, a cosmeceutical, or a personal care product.

One object of the disclosure is a colored composition where colorants and the clear or translucent composition are combined. The colorants may include traditional colorants, goniochromatic colorants, multi-dimensional pigments, pearlescents, and holographic glitters.

In a further object of the disclosure, the unique combination of components or ingredients produces a clear or translucent composition that is processed using existing cosmetic manufacturing equipment.

Yet another object relates to a method for the manufacture of a clear or translucent stick with a high stick strength.

In a further object, a method of producing the clear or translucent composition comprises mixing components (a), (b), (c), and (d), thereby producing a clear or translucent composition where the mixture of (a), (b), (c), and (d) has a dissolution temperature of about 100 °C or less and is affected in about 5 to about 10 minutes.

These and other objects of the present disclosure will become apparent to those skilled in the art after a reading of the following detailed description, including the illustrative embodiments and examples.

### DETAILED DESCRIPTION

The present disclosure is directed to a clear or translucent composition, methods of use, and methods of producing the clear or translucent composition thereof. The composition may take the form of a solid, semi-solid, or gel. Another embodiment relates to the clear or translucent composition and additional ingredients having desired properties.

A further embodiment is directed to the synergistic combination of known ingredients that is processed with existing cosmetic manufacturing equipment and results in the production of a strong free-standing stick composition. More specifically, the clear or translucent composition comprises:
(a) at least two amino acid - based gelatinizing agents;
(b) a non-ionic unsaturated fatty alcohol;
(c) an alkyl dimethicone; and
(d) an oil, a polymer, or combinations thereof.

One of the problems in the art of stick compositions is the lack of composition strength which is required to form a free-standing stick composition. Another problem occurs during manufacture and production. Without enough or sufficient stick strength, the composition is oftentimes damaged when ejected or removed from a mould, A further issue with the clear stick compositions of the art is its softness, which is softer than conventional wax-based stick compositions. The solution in the art has been to house the soft stick composition in a walled support style container, such as those found in a gel or stick deodorant or Chapstick® style stick packaging. Therefore, in order to overcome these problems in the art, the amount of amino acid - based gelatinizing agent is increased to a significant amount greater than what is found in the art, and one or more alkyl dimethicone materials is added to the clear or translucent composition of the disclosure.

As the skilled artisan understands, dissolution occurs when all of the ingredients or components of a composition completely dissolve. However, in order to achieve complete dissolution of the prior art compositions comprising amino acid - based gelling agents, the temperature must be elevated at or above 130 °C followed by a lengthy gelling time of at least 15 hours in order to cool the composition sufficiently in order to gel at a temperature of about 23 °C to about 25 °C. Although the amount of amino acid - based gelling agent(s) used in the prior art compositions is generally extremely small (1:100 gellants to oil ratio), the amino acid - based gellants must still be processed under extreme conditions for prolonged periods of time as stated above in order to achieve a gel. In particular, a temperature ranging from about 130 °C to over about 200 °C is typically required for those amino acid - based gelatinizing agents, as discussed above and found in the prior art, to go into solution. Because of the extremely high temperature required for dissolution, prior art compositions which require these gelatinizing agents were limited to a select group of oils or polymers which could withstand the extremely high temperatures.

Another disadvantage of using these gelatinizing agents is that the time for them to gel typically takes a very long time. For example, for the gels in the prior art to set, the required time and temperature would be about 15 hours at 25 °C. Therefore, processing these gelatinizing agents in the art utilizes a lot of energy due to the high dissolution temperature, reduces the amount of output in view of the lengthy time required to obtain the product, limits the oils or polymers that could be used, and increases the total manufacturing costs.

Whereas, the particular combination of ingredients or components of the composition of the present disclosure allows for dissolution at much lower temperatures and thereby requires a shorter amount of time to obtain the disclosed clear or translucent gel composition having improved properties over the art. As previously stated, the combination of the amino acid - based gellants of the disclosure, the unsaturated fatty alcohol, alkyl dimethicone, and oils or polymers, achieves a gel at or below 100°C in a matter of minutes to a few hours depending upon the overall batch size.

In another embodiment of the disclosure, the amino acid - based gelatinizing agents of the inventive composition comprise a mixture of at least two amino acid - based gelatinizing agents, three or more amino acid - based gelatinizing agents may also be useful. Non-limiting examples include any of the known gelatinizing agents, N-acetyl glutamic acid dilauryl amide, N-caproyl glutamic acid dibutyl amide, N-lauryl glutamic acid dihexyl amide, N-lauroyl-glutamic acid dihexyl amide, and N-acetyl amino acid amides. In a specific embodiment, the mixture of amino acid - based gelatinizing agents includes N-lauroyl-L-glutamic acid dibutyl amide and N-2-ethylhexanoyl-L-glutamic acid dibutyl amide. If there are two amino acid - based gelatinizing agents, then they are in a weight ratio of 1:3 to 3:1, where a preferred ratio is 1:1. However, in another embodiment where there are more than two amino acid -based gelatinizing agents, the weight ratio is, for example, 1:1:1.

Furthermore, the mixture of or total amount of amino acid - based gelatinizing agents is more than the aforementioned prior art gellants. Another embodiment of the disclosure is directed to a composition comprising amino acid - based gellants having a weight percent of about 1.5 % to about 5 % of the total composition, about 1.6 % to about 2.4 %, or about 2.25 % to about 3 % of the total composition, in contrast to the typically less stable stick compositions in the art which comprise amino acid - based gelatinizing agents less than 1 % by weight of the total composition. Moreover, less stable stick compositions in the art typically employ one or two amino acid - based gellant(s), and such compositions do not include alkyl dimethicone or derivatives thereof. The synergistic combination of the amino acid - based gellants and the alkyl dimethicone allows for the production of a more rigid, free-standing cosmetic sticks that are capable of withstanding extreme environmental conditions whilst maintaining their integrity. In the prior art, very high temperatures were necessary to effect dissolution of the amino acid - based gellants. However, dimethicone materials are not processed at such high temps. Furthermore, the dimethicone helps stabilize the stick in two ways: first, dimethicone makes the stick more rigid; and second, the dimethicone prevents the stick from "sweating." When compositions are exposed to freezing conditions or high temperatures, such as during storage or transportation, the compositions will sweat or exude solvent when returning to ambient conditions from either of the extreme temperatures listed. However, due to the unique combination of ingredients disclosed herein, sweating is not problematic.

Another embodiment of the disclosure is directed to the clear or translucent composition comprising at least one non-ionic unsaturated fatty alcohol which can dissolve the amino acid - based gelatinizing agents or gellants at or below a temperature of 100 °C. One or more of the non-ionic unsaturated fatty alcohols useful for dissolution includes but is not limited to a non-ionic mono- or polyunsaturated fatty alcohol. Non-limiting examples of useful non-ionic unsaturated fatty alcohols of the disclosure include oleyl alcohols, octyldodecanols, 2-butyloctanals, 2-hexyldecanols, and 2-undecylpentadecanols. A particular embodiment is directed to oleyl alcohol. Oleyl alcohol examples include, but are not limited to octadecenol and particularly, cis-9-octadecen-1-ol. The non-ionic unsaturated fatty alcohol of the inventive composition is present in an amount from about 0.1% to about 30 % by weight of the total composition. Other embodiments are directed to an amount of about 15 % to about 30 %, and further, about 16 % to about 25.5 % by weight of the total composition.

Selection of the fatty alcohol is dependant upon its ability to dissolve the amino acid - based gellants at or below 100 °C. In one embodiment, the useful unsaturated fatty alcohols are nonvolatile. Nonvolatile is defined herein as those fatty alcohols having at 1.0 atmosphere, a boiling point of at least about 200 °C, at least about 205 °C, and at least about 210 °C. Some fatty alcohols of the instant disclosure have, for example, one double bond (monounsaturated). Their general formula is: CH₃(CH₂)ₓ(CH=CH(CH₂)_{y}-CH₂OH.

Suitable fatty alcohols also include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, less than about 5 % by total weight of the unsaturated straight chain fatty alcohol, less than about 2%, or less than about 1 %. In certain embodiments, the unsaturated straight chain fatty alcohols have an aliphatic chain length ranging from C12 - C22, from C12 - C18, or from C16 - C18, such as, for example, oleyl alcohol and palmitoleic alcohol. Non-limiting examples of non-ionic unsaturated fatty alcohols include those identified in Table 1:

**TABLE 1**

| Common Name | Chemical Name | Molecular Structure | # Carbons |
|---|---|---|---|
| | | | |
| palmitoleyl alcohol | (cis-9-hexadecen-1-ol) | CH₃(CH₂)₅CH=CH(CH₂)₈OH | 16 |
| | | | |
| elaidyl alcohol | (9E-octadecen-1-ol) | CH₃(CH₂)₇CH=CH(CH₂)₆OH | 18 |
| | | | |
| oleyl alcohol | (cis-9-octadecen-1-ol) | CH₃(CH₂)₇CH=CH(CH₂)₈OH | 18 |
| | | | |
| linoleyl alcohol | (9Z, 12Z-octadecadien-1-ol) | polyunsaturated | 18 |
| | | | |
| elaidolinoleyl alcohol | (9E, 12E-octadecadien-1-ol) | polyunsaturated | 18 |
| | | | |
| linolenyl alcohol | (9Z, 12Z, 15Z-octadecatrien-1-ol) | polyunsaturated | 18 |
| | | | |
| elaidolinolenyl alcohol | (9E, 12E, 15-E-octadecatrien-1-ol) | polyunsaturated | 18 |
| | | | |
| ricinoleyl alcohol | (12-hydroxy-9-octadecen-1-ol) | polyunsaturated | 18 |
| | | | |
| ricinoleyl alcohol | (12-hydroxy-9-octadecen-1-ol) | CH₃(CH₂)₅CH(OH)CH₂CH=C H(CH₂)₈OH unsaturated, diol | 18 |
| | | | |

**TABLE 1**

| Common Name | Chemical Name | Molecular Structure | # Carbons |
|---|---|---|---|
| erucyl alcohol | (cis-13-docosen-1-ol) | CH₃(CH₂)₇CH=CH(CH₂)₁₂OH | 22 |
| | | | |

In another embodiment, the alkyl dimethicone component provides a thermally stable stick composition of the disclosure. In addition to improving the structure strength of the disclosed composition thereby easing removal of the composition from a mould, alkyl dimethicone also prevents stick compositions from "sweating" in elevated temperatures, for example, during storage or product transport. Clear stick compositions in the art tend to 'sweat' or 'weep' one or more solvents at elevated temperatures. These high temperatures are generally between about 130 °C and above 200 °C. In order to overcome these problems in the art, an alkyl dimethicone material (which relates to one or more alkyl dimethicones) may be included in the clear or translucent composition of the disclosure.

As is understood in the art, alkyl dimethicones can be either silicone- or oil- soluble, depending on the ratio of x, y and z in the below formula.

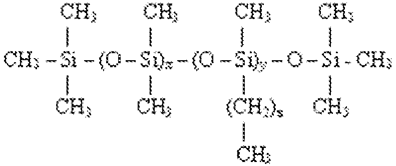

As the value of x and y increases, the alkyl dimethicone become harder and more soluble in silicone oil. As the z value increases, the alkyl dimethicone will be more soluble in oils, such as vegetable, mineral, and ester, which also raises the melting temperature of the waxes. As the value of x increases and the value of z decreases, the more soluble the alkyl dimethicone becomes in silicone. However, as the values of y and z increase, the alkyl dimethicone becomes more oil soluble.

As is well known by skilled artisans in the formulations art, varying the ratio of x, y and z determines whether alkyl dimethicone is a liquid, a soft paste or a hard wax. Generally, using a higher number of alkyl groups, for example 22 carbons, the product will be a paste or hard wax, depending on the ratio of x to y. A higher value of x lowers the melting point of the product. While, an olefin with 18 carbon groups, the alkyl dimethicone can be a liquid to hard wax, depending on the ratio of x to y. Alkyl groups with 16 carbon groups or lower are normally liquids to soft pastes. There has been found an improvement in SPF values when alkyl dimethicones are added to sunscreens. Therefore, one of ordinary skill in the art who will consider the factors related to the desired results, will determine the exact ratios of x, y, and z. The ratios are adjusted to provide structure or to maintain the desired effect. Factors, which may be taken into account, include the use and desired form of the final product.

A particular embodiment of the present disclosure is directed to an alkyl dimethicone, or a combination of compatible alkyl dimethicones, in an amount ranging from about 0.1 % to about 30 % by weight of the total composition, about 10 % to about 25 % by weight, and about 15 % to about 20 %. The alkyl dimethicone useful in the instant disclosure has an alkyl in the range of about 18 % to 65 % by weight of the entire alkyl dimethicone, while the dimethicone is in the corresponding range of about 35 % to about 82 % by weight of the alkyl dimethicone. In another embodiment, alkyl dimethicones containing from 1 to 18 carbons, such as, for example an alkyl dimethicone of stearyl dimethicone C18, are most useful. Additional non-limiting examples of alkyl dimethicones of the instant disclosure include behenyl dimethicone, C-32 alkyl dimethicone, isopropyl phenyl dimethicone, cerotyl dimethicone, hydroxypropyl diemthicone behenate, cetyl/hexacosyl dimethicone, and the D and J series of Silwax® alkyl dimethicones and Multi Domain™ silicones (Siltech LLC; Dacula, GA).

In yet a further embodiment, the oil or polymer of the clear or translucent composition of the disclosure may include either an oil or a polymer, but also may be a combination of multiple oils, multiple polymers or both, i.e., a combination of one or more oils and/or polymers. The oil or polymer of the disclosure includes, but is not limited to, non-polar oils, polar oils, liquid polymers, solution polymers, or combinations thereof. The oils used for the clear or translucent composition of the present disclosure that are suitable for purposes of this composition include those that sufficiently allow the dissolution of the gelatinizing agents upon heating and thereby resulting in the formation of a gel when cooled. Non-limiting examples include mineral oils, isohexadecane, jojoba oils, C10 - C30 cholesterol/ Lanoesterol esters, e.g. Super Sterol Liquid (Croda Inc.; Edison, NJ), mink oil, cacao oil, coconut oil, palm seed oil, camellia oil, sesame oil, castor oil, olive oil, aloe extract, and silicone oil.

More specifically, illustrative ester oils include esters such as tridecyl trimellitate., triisostearyl citrate, diisostearate maleate, diisostearyl fumarate, and the like. Liquid polymers or solution polymers useful in the present disclosure include, but are not limited to, polybutene, hydrogenated polyisobutene, triisostearyl polyglyceryl-3-dimer dilinoleate, polyglycerol diisostearate, and, such as. The oil or polymer component of the composition disclosed herein may be in an amount ranging from about 30 % to about 70 % by weight of the total composition, more particularly, ranging from about 32.5 % to about 47.5 % of the total composition, or about 42 % to about 65 % of the total composition.

Without being bound by theory, in order to obtain a clear or translucent composition that has the benefit of a relatively fast dissolution and gelling time, as well as low dissolution and gelling temperature, that has relatively increased stick strength, and reduced sweating properties, the non-ionic, unsaturated fatty alcohol, such as oleyl alcohol, that is compatible with the amino acid - based gelatinizing agents, in particular, N-lauryol-L-glutamic acid dibutylamide and N-2-ethyl-hexanoyl-L-glutanic acid dibutylamide in a weight ratio of 1:3 to 3:1, or more specifically, 1:1, provides complete dissolution at or below 100 °C and in a matter of minutes.

In yet another embodiment, the clear or translucent composition comprises additional components. The clear or translucent composition additionally comprises an antioxidant to protect the non-ionic unsaturated fatty alcohol from degrading. In a particular embodiment, one or more antioxidants may be used in an amount ranging from about 0.01 % to about 0.5 % by weight of the total composition. One anti-oxidant useful in the clear or translucent composition of the disclosure is a butylated hydroxyl toluene (BHT). However, any antioxidant compatible with the other components of the inventive composition may be used, including but not limited to potassium sulfite, sodium bisulfite, butylated hydroxyanisole, and the like. The antioxidant may be in an effective amount to protect the fatty alcohol from degrading, such as for example, between about 0.01 % to about 0.5 % by weight of the total composition, about 0.06 % to about 0.5 %, and about 0.3 % to about 0.5 % by weight. Examples 1 and 2 provide exemplary clear or translucent formulations.

Traditional stick compositions employ high melting point waxes, such as paraffin wax, beeswax, carnauba wax, ozokerite wax, microcrystalline, and polyethylene wax are used in order to provide the composition with structure. Such waxes have high melting points and create a matrix where the structure is comprised of randomly orientated crystals resulting in an opaque appearance. As a result, these waxes interfere with the colorants and the resulting visualized color is not accurate. Therefore, in another embodiment of the present disclosure, the clear or translucent composition combined with either traditional colorants and/ or goniochromatic colorants delivers a true color" with a vibrancy only observed thus far in clear liquid media.

In embodiments where the inventive composition is a color cosmetic, such as but not limited to, a lipstick or lip gloss and the like, the compositions further comprise one or more coloring agents. It is within the skill in the art to select coloring agents and combinations of coloring agents to produce a desired color or effect. Suitable traditional coloring agents, including pigments, lakes, and dyes, are well known in the art and are disclosed in the C.T.F.A. Cosmetic Ingredient Handbook, First Edition, 1988, the contents or which are hereby incorporated by reference. Organic pigments include, for example, FD&C dyes, D&C dyes, including D&C Red, Nos. 2, 5, 6. 7, 10, 11, 12, 13, 30 and 34, D&C Yellow No. 5, Blue No. 1, Violet No. 2. Exemplary inorganic pigments include, but are not limited to, metal oxides and metal hydroxides such as magnesium oxide, magnesium hydroxide, calcium oxide, calcium hydroxides, aluminum oxide, aluminum hydroxide, iron oxides (α-Fe₂O₃, γ-Fe₂O₃, Fe₃O₄, FeO), red iron oxide, yellow iron oxide, black iron oxide, iron hydroxides, titanium dioxide, titanium lower oxides, zirconium oxides, chromium oxides, chromium hydroxides, manganese oxides, cobalt oxides, cerium oxides, nickel oxides and zinc oxides and composite oxides and composite hydroxides such as iron titanate, cobalt titanate and cobalt aluminate. Other suitable coloring agents include ultramarine blue (*i*.*e*., sodium aluminum silicate containing sulfur), Prussian blue, manganese violet, bismuth oxychloride, talc, mica, sericite, magnesium carbonate, calcium carbonate, magnesium silicate, aluminum magnesium silicate, silica, titanated mica, iron oxide titanated mica, bismuth oxychloride, and the like. The coloring agents may be surface modified, with, for example, fluoropolymers, to adjust one or more characteristics of the coloring agent as described in, for example, U.S. Patent Nos. 6,471,950, 5,482,547, and 4,832,944, the contents of which are hereby incorporated by reference. Fluoropolymers may be incorporated into the present disclosure as a coating on pigment particles that at least partially covers the surface of the pigment particles. Suitable pearling pigments include without limitation bismuth oxychloride, guanine and titanium composite materials containing, as a titanium component, titanium dioxide, titanium lower oxides or titanium oxynitride, as disclosed in U.S. Patent No. 5,340,569, the contents of which are hereby incorporated by reference.

In one embodiment of the disclosure, traditional colorants are employed in an amount ranging from about 0.1 % to about 25 % by weight of the total composition. The combination of any such colorant with the herein disclosed clear or translucent composition delivers a true color rarely seen in traditional solid formulations. In yet another embodiment of the disclosure, the goniochromatic colorants useful in the inventive compositions described herein is in an amount ranging from about 0.1 % to about 15 % by weight of the total composition. The goniochromatic colorants, also known as color travel pigments, include platelet-shaped base substrates coated with alternating layers of high or low refractive index materials. Goniochromatic colorants are pigments that exhibit different colors depending on the viewing angle.

Illustrative colorants include Reflecks™ MultiDimensions (BASF Corp.; Florham Park, NJ) pigments that can shift across the entire visual spectrum to provide multiple color effects through its borosilicate pigment technology, and SpectraFX which is a powdered flake additive available in a variety of sizes from very small to extra large (Alsa Corp.: Vernon, CA). Reflecks™ MultiDimensions pigments are composed of a calcium sodium borosilicate base coated with silica, titanium dioxide, and tin oxide. In a particular embodiment of the disclosure, goniochromatic colorants were added to the clear or translucent composition to deliver unique color effects. These goniochromatic colorants range in particle size from about 1 micrometer to about 100 micrometers, or more specifically, from about 40 micrometers to about 80 micrometers, as exemplified with SpectraFX pigments and Reflecks™ pigments, respectively.

A further embodiment is directed to the amount of goniochromatic colorant useful in the clear or translucent composition of the disclosure, which ranges from about 0.1 % to about 15 % by weight of the total composition, while in a specific embodiment, the amount of colorant ranges from about 0.5 % to about 5 % by weight of the total composition. The particle size and amount of colorant used in the composition determines the final optical effect of the composition. As is understood by the person of ordinary skill in the art, a weight percent load of 0.5 % of the Copper/Patina SpectraFX pigment results in translucency as well as a perceived color shift from copper/ patina to green depending on the viewing angle. Whereas, at higher colorant loadings, the translucency of the composition is diminished due to the fact that less light may travel through the composition. However, there is still a perceived color shift depending upon the viewing angle.

Similarly, in yet a further embodiment, combined with the clear or translucent composition, pearlescent type colorants may be used to deliver better or more true color effects than seen in a traditional opaque, wax-based stick composition. Another embodiment is directed to the clear or translucent composition with holographic glitters that produce unique effects. These glitters include those that are holographically embossed, vacuum metalized polyester flakes, such as those available from Spectratek Technologies, Inc. (Los Angeles, CA). In the same way that the visual effects of the SpectraFX pigments varies depending on the load amount, the same holds true for the holographic glitters. When added at a load of less than about 2 % by weight of the total composition, the inventive composition combined with the holographic glitters produce a translucent appearance, and the glitters look like they are suspended within the composition, such that different colors are produced by the different glitter particles. However, at higher colorant loads, the translucency is diminished.

In yet a further embodiment, the clear or translucent composition is combined with a mixture of traditional colorants and goniochromatic colorants, *i*.*e*., not a dual core style as described above. When the inventive composition is used in such a manner, the typical amount of traditional colorant ranges from about 0.1 % to about 25 % by weight of the total composition and the goniochromatic colorant ranges from about 0.1 % to about 15 % of the total composition.

Another embodiment is directed to a method of making the clear or translucent composition as described herein. One embodiment which utilizes the traditionally used machinery, comprises simultaneously mixing all of the components, including:
(a) at least two amino acid - based gelatinizing agents;
(b) a non-ionic unsaturated fatty alcohol;
(c) an alkyl dimethicone component; and
(d) an oil, a polymer, or combinations thereof,
at a temperature of about 100 °C or lower sufficient to dissolve the components. Typically dissolution occurs within minutes to hours depend on batch size. The gel temperature useful in the instant disclosure ranges from about 25 °C to about 80 °C, from about 65 °C to about 80°C, or in the most useful embodiment, around room temperature or about 25 °C. Once again, the time necessary for the clear or translucent composition to gel is on the scale of minutes and not hours. Example 2 is directed to an example of a method of producing the clear or translucent composition of the instant disclosure.

In yet a further embodiment of the disclosure, the method is directed to mixing components (a) and (b) first, heating the mixture of (a) and (b) to a temperature of at most about 100 °C or lower thus dissolving the mixture. Subsequently, components (c) and (d) are added either simultaneously or subsequently of each other, and dissolved at a similar temperature, *i*.*e*., no more than about 100 °C. The resultant mixture of components (a), (b), (c), and (d) is then cooled to room temperature (*i*.*e*., 25 °C) to form a viscous gel.

Another embodiment of the disclosure is directed to the addition of optional ingredients that contribute other benefits to the inventive composition. Non-limiting active or functional ingredients may include colorants, pigments, ultraviolet filters, moisturizing agents, fragrances, insecticides, pesticides, pharmaceutical agents, and other active or functional ingredients known in the cosmetic or pharmaceutical arts. These additional ingredients may be mixed simultaneously with the components of (a), (b), (c), and (d), or with components (c) and (d), or subsequent to the mixture of any or all of the components of (a), (b), (c), or (d). Regardless, the additional ingredients must be compatible with the parameters of the inventive composition, *i.e.*, for example, dissolution at a temperature at or below about 100 °C. A further embodiment is directed to any of the aforementioned methods comprising an additional step of casting the resultant mixture or viscous gel into a mould.

Accordingly, the present disclosure relates to a clear or translucent composition that may have numerous applications. In particular, the inventive composition may be a cosmetic product, uncolored or colored, that is particularly useful for application to the face, lips, eyes, cheeks and body. In one embodiment, the clear or translucent composition is a stick composition, such as, for example, those used in primers, foundations, eye products, lip products, sunblocks or sunscreens, anti-perspirants, anti-deodorants, and the like. Although solid products are widely used in cosmetics, the present disclosure is unique in that the gel forming step is performed at significantly lower temperatures through the use of an unsaturated fatty alcohol and the further addition of an alkyl dimethicone material which results in a product in the form of a solid, semi-solid or viscous gel. The resulting composition is thermally stable beyond any prior art that employs one or more amino acid - based gellants. Depending on the particular use of the clear or translucent composition, additional ingredients may be included.

Another embodiment of the instant disclosure relates to a combination of the clear or translucent composition as described herein with a traditional colored lipstick composition as separate entities, to create a hybrid lip product. In another embodiment of the present disclosure, the clear or translucent composition may be combined with either a traditional wax based lipstick or the inventive composition of the disclosure containing colorants. This combination may be presented where for example, in a cylindrical lipstick composition, from the top view looking down, half of the composition is the traditional lipstick while the other half is the inventive clear or translucent composition (with or without colorants). Another embodiment is directed to a dual core style configuration. For example, the inventive clear or translucent composition may form an outer shell and the traditional lipstick forms an inner core such that the outer shell surrounds the inner core which is visible through the clear or translucent outer shell forming concentric circles. Similarly, the colored inventive composition may form the inner core, such that even though the clear or translucent outer shell surrounds the inner core, the colors from the inner core are visible. Infinite color combinations are possible, in addition special color effects are possible by incorporating goniochromatic pigments in the outer, clear shell such that they are complimentary to the inner core coloration, or in certain embodiments, the colorant is in the inner core. The loading of colorant in the different concentric portions is dictated by the desired end visual effect as applied or as stored.

A further embodiment of the disclosure relates to a composition, preferably a topical solid stick, semi-solid, or gel, that is a cosmetic, a personal care product, a cosmeceutical or medicinal formulation, an insect repellent, or a sun product, where the composition comprises a non-ionic unsaturated fatty alcohol that sufficiently dissolves the other components, such as for example, at least two amino acid - based gelatinizing agents, oils, polymers, antioxidants, fragrances, and the like. The disclosed composition may be used in products, such as but not limited to, color cosmetics, sun care, skin care, hair products (shampoos, conditioners, hairspray, mousses and dyes/colorants), a mascara, a nail enamel, a lip coloring product, a foundation, eye make-up, a skin care product, a personal hygiene product, and a topical drug or active delivery.

In another embodiment, the composition of the disclosure may also include any additive usually employed in the field envisaged such as antioxidants, perfumes, essential oils, stabilizers, cosmetic active substances, moisturizers, vitamins, essential fatty acids, lipophilic sunscreens, liposoluble polymers, and especially hydrocarbon polymers such as polyalkylenes and polyacrylates for improving smoothness or spreadability, water and oil resistance, transfer resistance, or other cosmetic or cosmeceutical properties desired by one of skill in the art. Non-limiting examples of optionally added ingredients include: emollients, thickening agents, for example, opacifying agents, clays, or organoclays, silicas, cellulose derivatives, plasticizers, gels, fatty acids, fats, powders, oils, preservatives, solvents, surfactants; hectorites; synthetic polymers such as an acrylic polymer or an associative polymer of the polyurethane type; gums and in particular xanthan gum; spreading agents; dispersants; preservatives, in particular water-soluble preservatives; antifoaming agents; wetting agents; ultraviolet-screening agents; perfumes; fillers and bulking agents; binders; cosmetic or pharmaceutical active agents; moisturizers; feel enhancers including but not limited to powders and oils; vitamins and derivatives thereof; and biological materials and derivatives thereof. If the softness and elasticity of the composition are to be increased still further, it is also possible to add a plasticizer which is commonly added for cosmetic materials. Suitable materials may include both low-molecular weight and also high-molecular weight plasticizers which are optionally used, solubilized, or dissolved in a cosolvent.

Suspending and thickening agents typically include silica gels, gums, clays, fumed silica, fatty acid soaps, and various hydrocarbon gels, and other ingredients that when incorporated into the formulation remain on the surface of keratinous tissues. Non-limiting examples of ingredients, such as emollients, that may preferably be used in the compositions of the disclosure include glycerine, propylene glycol, cyclomethicone, dimethicone, and emollients and other similar ingredients disclosed in the International Cosmetic Dictionary and Handbook Vols. 1 and 2. Eds. Wenninger, J.A. and G.N. McEwen, Cosmetic, Toiletry, and Fragrance Association, Washington DC, 2000, which is hereby incorporated by reference.

A pigment that is not necessarily one of the goniochromatic pigments described above, should be understood to mean inorganic or organic, white or colored particles. Coloring agents that may be used in the practice of the disclosure may include pigments, lakes, and dyes which are well known in the art and are disclosed in the Cosmetic Ingredient Handbook, First Edition, J.M. Nikitakis, et al., Cosmetic, Toiletry, and Fragrance Association, Washington DC, 1988, the contents or which are hereby incorporated by reference. Depending on the application for the composition, pigments may be added to provide color or no color.

Other embodiments may be directed to the inventive compositions with fillers, mother-of-pearl, and the like, to modify the texture of the composition and the matte or glossy appearance. Fillers should be understood to mean lamellar or nonlamellar, inorganic or synthetic, colorless or white particles. Mother-of pearl should be understood to mean iridescent particles produced especially by certain mollusks in their shell or else synthesized. Pearling agents that may be used in the practice of the disclosure include mica, iron oxides, titanium dioxide and any other pearling agent known in the cosmetic arts. Non-limiting examples of fillers and microspheres used either alone or in combination, for example, the pressed powder composition prototypes include: talc, corn starch nylon powder, polymethyl methacrylate, polytetraflourothylene, zinc stearate, boron nitride, calcium silicate, and the like.

Compounds commonly used in the cosmetic arts for preventing or reducing fungal, bacterial, or microorganismal growth are also added to the composition of the disclosure. By including these compounds, the shelf life of the composition is lengthened. These anti-fungal and anti-microorganisms include but are not limited to methyl paraben, butyl paraben, sodium dehydroacetate, etc. The amounts of these anti-fungal or anti-microorganism ingredients are in an amount effective to reduce growth without negatively affecting the components of the inventive composition or the desired effects.

The person skilled in the art will of course take care to choose the optional additional compounds and/or their quantities in such a way that the advantageous properties of the composition according to the disclosure are not, or are substantially not, impaired by the envisaged addition(s). In embodiments where these materials are added to the formulations of the disclosure to enhance the spreadability and the emollience of the product, however, it is preferred that the above materials be present in low enough concentrations for the formulation to retain its desired properties. These ingredients may be selected variously by the person skilled in the art in order to prepare a composition which has the desired properties, for example, composition strength, true color optical effect, and dissolution temperature of about 100 °C or less on the order of within minutes versus hours. The choice of additional ingredients and their concentrations may also be adjusted to vary the desired properties. In one embodiment, a fragrance may be added for superficial purposes, such as for example, to make the inventive composition more appealing to the consumer.

This technology and the inventive compositions are applicable to a wide variety anhydrous and powdered products, including but not limited to: foundations, concealers, mascaras, blushes, eyeliners, eyeshadows, face or body powders, as well as skin care products, such as sun screens and insect repellants. In particular, the composition of the disclosure may include a cosmetic formulation. One embodiment of the disclosure relates to cosmetic foundations, where the formulation of a cosmetic foundation may contain, in addition to the composition of the disclosure, additional thickening agents and emollients in an amount that provides coverage and achieves the other desired properties.

A further embodiment of the disclosure includes lotions or stick compositions including reflectors, absorbers, or filters of ultraviolet rays to provide a suntan lotion or sunblock. A convenient means of carrying a sunblock is in a solid stick formulation. The stick formulation avoids the disadvantages of a lotion.

Yet another embodiment of the disclosure includes eyeliner and eyeshadow products. For example, eyeliners and eyeshadows employing the composition of the disclosure may provide increased color variability when the disclosed composition is presented with goniochromatic pigments.

The packaging of the inventive composition into, for example, a kit or article of manufacture, and application device for any embodiment of the disclosure is chosen and manufactured by persons skilled in the art on the basis of their general knowledge, and adapted according to the nature of the composition to be packaged. Moreover, the type of device to be used may be in particular linked to the consistency of the composition, in particular to its viscosity; it may also depend on the nature of the constituents present in the composition, such as the presence of volatile compounds. The kit or article of manufacture may include, but is not limited to, the inventive composition, a device for the application of the inventive composition, instructions for the use and application of the inventive composition, a listing of ingredients and/or warnings, and the like. A preferred article of manufacture is one that presents a stick composition.

### EXAMPLE

The following example further describes and demonstrates embodiments within the scope of the present disclosure. The example is given solely for the purpose of illustration and is not to be constructed as limitations of the present disclosure, as many variations thereof are possible without departing from the spirit and scope of the disclosure. The following exemplary composition is given by way of illustration only and in no way intended to be limiting to the scope of disclosure.

### EXAMPLE 1

### CLEAR OR TRANSLUCENT STICK COMPOSITION

All of the ingredients identified in Table 2 were mixed using a rotor-stator high shear equipment at a speed of between about 500 and 10,000 rpm at a temperature of about 100 °C for about 5 to about 15 minutes or until a clear solution was obtained. Although a "regular" mixer using low shear could be used, the process would take much longer. The clear solution was poured into a mould to form a clear or translucent stick composition.

**TABLE 2**

| Ingredients | Weight Percent (%) |
|---|---|
| EB-21 | 2.25 |
| GP-1 | 2.25 |
| oleyl alcohol | 25.50 |
| hydrogenated polyisobutene | 23.75 |
| hydrogenated polyisobutene gel | 23.75 |
| stearyl dimethicone | 15.00 |
| BHT | 0.50 |
| ethylhexyl methoxycinamate | 7.00 |
| | |

### EXAMPLE 2

### CLEAR OR TRANSLUCENT STICK COMPOSITION

All of the ingredients identified in Table 3 were mixed using a rotor-stator high shear equipment at a speed of between about 500 and 10,000 rpm at a temperature of about 100 °C for about 5 to about 15 minutes or until a clear solution was obtained. The clear solution was poured into a mould to form a clear or translucent stick composition.

**TABLE 3**

| Ingredients | Weight Percent (%) |
|---|---|
| EB-21 | 25.50 |
| GP-1 | 2.25 |
| oleyl alcohol | 2.25 |
| hydrogenated polyisobutene | 16.25 |
| hydrogenated polyisobutene gel | 16.25 |
| stearyl dimethicone | 30.00 |
| BHT | 0.50 |
| ethylhexyl methoxycinamate | 7.00 |
| | |

### EXAMPLE 3

### METHOD OF PREPARING A CLEAR OR TRANSLUCENT COMPOSITION

In order to obtain a clear or translucent composition, the components were combined such that the dissolution temperature and gel temperature were lower than what is commonly found or expected based on the characteristics of the individual components. The time for the components to dissolve and form a gel was also much shorter, on the scale of minutes to hours (batch size from 100 grams to 2 kilograms).

A preferred method and composition is described herein. The method is a two step process. The following amino acid - based gelatinizing agents and non-ionic unsaturated fatty alcohol ingredients of Table 4 were mixed together using rotor-stator high shear equipment.

**TABLE 4**

| Ingredients | Weight Percent (%) |
|---|---|
| N-lauroyl-L-glutamic dibutylamide | 8.0 |
| N-2-ethylhexanoyl-L-glutamic acid dibutylamide | 8.0 |
| oleyl alcohol | 84 |

The mixture of Table 4 was heated to a temperature of 100 °C for about 5 to about 15 minutes, or until a clear solution is obtained. The resulting gel concentrate was combined with the preheated mixture of oils or polymers, as well as additional optional ingredients, such as an antioxidant, a sunscreen, and a fragrance as indicated in Table 5 below.

**TABLE 5**

| Ingredient | Weight Percent (%) |
|---|---|
| Gel Concentrate of Table 2 | 20 - 30 |
| Alkyl dimethicone | 10 - 25 |
| Oil | 40 - 70 |
| Antioxidant | 0.01 - 0.5 |
| Sunscreen | 5 - 7 |
| Fragrance | 0 - 0.5 |

The resulting molten formulation was cast into a mould and cooled to a gel temperature of about -10 °C to about 25 °C to form a clear or translucent free standing stick composition of the disclosure.

## Claims

1. A clear or translucent composition, comprising: (a)N-lauryol-L-glutamic acid dibutylamide and N-2-ethyl-hexanoyl-L-glutanic acid dibutylamide; (b) oleyl alcohol; (c) stearyl dimethicone; and (d) an oil, a polymer, or combinations thereof, the oleyl alcohol and the - N-lauryol-L-glutamic acid dibutylamide and N-2-ethyl-hexanoyl-L-glutamic acid dibutylamide - based gelatinizing agents being present in a weight ratio of 1:3 to 3:1, and wherein the oleyl alcohol is present in an amount ranging from about 0.1 weight % to about 30 weight % of the total composition, wherein said composition has a dissolution temperature at or below about 100 °C.

2. The composition of claim 1, wherein said oil or polymer is: a polar oil, a non-polar oil, a liquid polymer, a solution polymer, or combinations thereof.

3. The composition of claim 2, wherein said oil or polymer is: a mineral oil, an isohexadecane, a squalene, a vegetable oil, a jojoba oil, a castor oil, an aloe extract, a lanolin oil, a C10-C30 cholesterol, a lanoesterol ester, a mink oil, a cacao oil, coconut oil, palm seed oil, camellia oil, sesame oil, a castor oil, olive oil, a silicone oil, a tridecyl trimellitate, triisostearyl citrate, diisostearate maleate, diisostearyl fumarate, a polybutene, a hydrogenated polyisobutene, a triisostearyl polyglyceryl-3-dimer dilinoleate, a polyglycerol diisostearate, or combinations thereof.

4. The composition of claim 1, further comprising at least one antioxidant.

5. The composition of claim 1, further comprising a colorant.

6. The composition of claim 5, wherein said colorant is: a goniochromatic colorant, an aluminum salt, a pearlescent, or glitter.

7. The composition of claim 6, wherein said goniochromatic colorant is present in an amount ranging from about 0.5 weight % to about 5.0 weight % of the total composition.

8. The composition of claim 6, wherein said glitter is holographically embossed, vacuum metalized polyester flakes.

9. A method, comprising: mixing components: (A) N-lauryol-L-glutamic acid dibutylamide and N-2-ethyl-hexanoyl-L-glutanic acid dibutylamide; (b) oleyl alcohol; (c) stearyl dimehicone; and (d) an oil, a polymer, or combinations thereof, the oleyl alcohol and the - N-lauryol-L-glutamic acid dibutylamide and N-2-ethyl-hexanoyl-L-glutamic acid dibutylamide - based gelatinizing agents being present in a weight ratio of 1:3 to 3:1, and wherein the oleyl alcohol is present in an amount ranging from about 0.1 weight % to about 30 weight % of the total composition, wherein said composition has a dissolution temperature at or below about 100 °C thereby producing a clear or translucent composition.

10. The method of claim 9, wherein components (a) and (b) are first mixed and heated at about 100.degree. C. or lower followed by the addition of components (c) and (d).

11. The method of claim 9, further comprising casting said dissolved components into a mould.

## Patentansprüche

1. Klare oder durchsichtige Lösung, umfassend: (a) N-Lauryol-L-glutaminsäure-dibutylamid und N-2-Ethyl-hexanoyl-L-glutaminsäure-dibutylamid; (b) Oleylalkohol; (c) Stearyldimethicon; und (d) ein Öl, ein Polymer oder Kombinationen davon, wobei der Oleylalkohol und die auf N-Lauryol-L-glutaminsäure-dibutylamid und N-2-Ethyl-hexanoyl-L-glutaminsäure-dibutylamid basierenden Geliermittel in einem Gewichtsverhältnis von 1:3 bis 3:1 vorhanden sind und wobei der Oleylalkohol in einer Menge im Bereich von etwa 0,1 Gew.-% bis etwa 30 Gew.-% der Gesamtzusammensetzung vorhanden ist, wobei die Zusammensetzung eine Lösungstemperatur von oder unter etwa 100°C aufweist.

2. Zusammensetzung nach Anspruch 1, wobei es sich bei dem Öl oder Polymer um Folgendes handelt: ein polares Öl, ein nichtpolares Öl, ein flüssiges Polymer, ein Lösungspolymer oder Kombinationen davon.

3. Zusammensetzung nach Anspruch 2, wobei es sich bei dem Öl oder Polymer um Folgendes handelt: ein Mineralöl, ein Isohexadecan, ein Squalen, ein Pflanzenöl, ein Jojobaöl, ein Rizinusöl, ein Aloe-Extrakt, ein Lanolinöl, ein C₁₀-C₃₀-Cholesterin, ein Lanoesterolester, ein Nerzöl, ein Kakaoöl, Kokosnussöl, Palmkernöl, Kamelienöl, Sesamöl, ein Rizinusöl, Olivenöl, ein Silikonöl, ein Tridecyltrimellitat, Triisostearylcitrat, Diisostearatmaleat, Diisostearylfumarat, ein Polybuten, ein hydriertes Polyisobuten, ein Triisostearyl-polyglyceryl-3-dimer-dilinoleat, ein Polyglycerol-diisostearat oder Kombinationen davon.

4. Zusammensetzung nach Anspruch 1, ferner umfassend wenigstens ein Antioxidationsmittel.

5. Zusammensetzung nach Anspruch 1, ferner umfassend einen Farbstoff.

6. Zusammensetzung nach Anspruch 5, wobei es sich bei dem Farbstoff um einen goniochromatischen Farbstoff, ein Aluminiumsalz, ein Perlglanzmittel oder Glitter handelt.

7. Zusammensetzung nach Anspruch 6, wobei der goniochromatische Farbstoff in einer Menge im Bereich von etwa 0,5 Gew.-% bis etwa 5,0 Gew.-% der Gesamtzusammensetzung vorhanden ist.

8. Zusammensetzung nach Anspruch 6, wobei es sich bei dem Glitter um holografisch geprägte, vakuummetallisierte Polyesterflocken handelt.

9. Verfahren, umfassend das Mischen der folgenden Komponenten: (a) N-Lauryol-L-glutaminsäure-dibutylamid und N-2-Ethyl-hexanoyl-L-glutaminsäure-dibutylamid; (b) Oleylalkohol; (c) Stearyldimethicon; und (d) ein Öl, ein Polymer oder Kombinationen davon, wobei der Oleylalkohol und die auf N-Lauryol-L-glutaminsäure-dibutylamid und N-2-Ethyl-hexanoyl-L-glutaminsäure-dibutylamid basierenden Geliermittel in einem Gewichtsverhältnis von 1:3 bis 3:1 vorhanden sind und wobei der Oleylalkohol in einer Menge im Bereich von etwa 0,1 Gew.-% bis etwa 30 Gew.-% der Gesamtzusammensetzung vorhanden ist, wobei die Zusammensetzung eine Lösungstemperatur von oder unter etwa 100°C aufweist, wodurch eine klare oder durchsichtige Zusammensetzung bereitgestellt wird.

10. Verfahren nach Anspruch 9, wobei die Komponenten (a) und (b) zuerst gemischt und auf etwa 100°C oder weniger erhitzt werden, worauf die Zugabe der Komponenten (c) und (d) folgt.

11. Verfahren nach Anspruch 9, ferner umfassend das Gießen der gelösten Komponenten in eine Form.

## Revendications

1. Composition transparente ou translucide, comprenant : (a) du dibutylamide d'acide N-lauroyl-L-glutamique et du dibutylamide d'acide N-2-éthyl-hexanoyl-L-glutamique, (b) de l'alcool oléylique, (c) de la stéaryldiméthicone et (d) une huile, un polymère ou des combinaisons de ceux-ci, dans laquelle l'alcool oléylique et les gélifiants à base de dibutylamide d'acide N-lauroyl-L-glutamique et de dibutylamide d'acide N-2-éthyl-hexanoyl-L-glutamique sont présents dans un rapport de poids de 1 pour 3 à 3 pour 1 et l'alcool oléylique est présent dans une proportion de 0,1 % du poids à environ 30 % du poids de la composition totale, ladite composition ayant une température de dissolution égale ou supérieure à 100 °C.

2. Composition selon la revendication 1, dans laquelle ladite huile ou ledit polymère est une huile polaire, une huile non polaire, un polymère liquide, un polymère en solution ou des combinaisons de ceux-ci.

3. Composition selon la revendication 2, dans laquelle ladite huile ou ledit polymère est une huile minérale, un isohexadécane, un squalène, une huile végétale, une huile de jojoba, une huile de ricin, un extrait d'aloès, une huile de lanoline, un cholestérol en C₁₀-C₃₀, un ester de lanostérol, une huile de vison, une huile de cacao, de l'huile de coco, de l'huile de palme, de l'huile de camélia, de l'huile de sésame, une huile de ricin, de l'huile d'olive, une huile de silicone, du trimellitate de tridécyle, du citrate de triisostéaryle, du maléate de diisostéaryle, du fumarate de diisostéaryle, un polybutène, un polyisobutène hydrogéné, du dilinoléate de triisostéaryl-polyglycéry1-3-dimère, un diisostéarate de polyglycérol ou des combinaisons de ceux-ci.

4. Composition selon la revendication 1, comprenant en outre au moins un antioxydant.

5. Composition selon la revendication 1, comprenant en outre un colorant.

6. Composition selon la revendication 5, dans laquelle ledit colorant est : un colorant goniochromatique, un sel d'aluminium, un pigment nacré ou des paillettes.

7. Composition selon la revendication 6, dans laquelle ledit colorant goniochromatique est présent dans une proportion d'environ 0,5 % du poids à environ 5,0 % du poids de la composition totale.

8. Composition selon la revendication 6, dans laquelle lesdites paillettes sont des flocons de polyester métallisés sous vide et gravés avec un hologramme.

9. Procédé comprenant : le mélange de (a) dibutylamide d'acide N-lauroyl-L-glutamique et dibutylamide d'acide N-2-éthyl-hexanoyl-L-glutamique, (b) alcool oléylique, (c) stéaryldiméthicone et (d) une huile, un polymère ou des combinaisons de ceux-ci, dans lequel l'alcool oléylique et les agents gélifiants à base de dibutylamide d'acide N-lauroyl-L-glutamique et de dibutylamide d'acide N-2-éthyl-hexanoyl-L-glutamique sont présents dans un rapport de poids de 1 pour 3 à 3 pour 1 et dans lequel l'alcool oléylique est présent dans une proportion de 0,1 % du poids à environ 30 % du poids de la composition totale, ladite composition ayant une température de dissolution égale ou supérieure à 100 °C, pour produire une solution transparente ou translucide.

10. Procédé selon la revendication 9, dans lequel les composants (a) et (b) sont d'abord mélangés à 100 °C environ ou moins, après quoi les composants (c) et (d) sont ajoutés.

11. Procédé selon la revendication 9, comprenant en outre le coulage desdits composants dissous dans un moule.
